# EUROPEAN PATENT APPLICATION

(11) **EP 0 775 504 A2**
(43) Date of publication of application: **28.05.1997**
(21) Application number: 96308444.7
(22) Date of filing: 21.11.1996
(51) Int. Cl.: A61N 5/06

(54) **Therapeutic lamp apparatus**

(30) Priority: 22.11.1995 GB 9523841
(71) Applicant: Spectron Laser Systems Limited, Rugby, Warwickshire CV21 1PB (GB)
(72) Inventor: Sarkies, Paul Harold, Rugby, Warwickshire CV22 6HP (GB)
(74) Representative: Hepworth, John Malcolm

(57) **Abstract**

The apparatus (Figure 1) comprises a lamp (8) mounted in a housing (2) so as to delivery light via an exit aperture (3) of the housing. A light guide member (15) is mounted in the housing and comprises a block of fused silica having an input face (16) adjacent to the lamp and an output face at the exist aperture. The light guide member is tapered in cross section towards the output face to concentrate light from the lamp and the side faces, (18, 19) of the light guide member are externally coated with a reflective coating (20). The apparatus is useful in therapeutic of human or animal skin, the solid light guide member preventing ingress of contaminants and providing safety in the event of lamp failure.

## Description

This invention relates to apparatus comprising a lamp for use in therapeutic treatment of human or animal body and in particular but not exclusively to a hand held apparatus operable to deliver pulsed light from a xenon flash lamp on to an area of skin contacted by the apparatus.

It is known from US 5405368 to provide apparatus in which a flash lamp is located in a housing having a reflector which directs light through an exit aperture on to an area of skin. A disadvantage of such apparatus is that the internal reflective surfaces of the reflector may deteriorate in use due to the high operating temperature of the lamp, the internal surfaces of the apparatus also being susceptible to contamination due to matter entering the apparatus via the exit aperture.

According to the present invention there is disclosed apparatus for use in therapeutic skin treatment comprising a housing defining an exit aperture, an arc lamp mounted in the housing and operable to deliver light via the exit aperture, a light guide member mounted in the housing and comprising a block of light conductive material having an input face disposed adjacent to the lamp and an output face at the exit aperture whereby in use an area of skin presented to the output face receives light from the lamp.

An advantage of such apparatus is that the light guide member, being formed of a block of material, prevents any ingress of contaminants. A further advantage is that, in the event of lamp failure accompanied by fragmentation of the lamp material, fragments of the lamp are prevented from reaching the exit aperture and are thereby prevented from causing any damage to the skin.

Preferably the light guide member is tapered in cross section towards the output face such that in use light from the lamp is concentrated before emerging from the output face.

The angle of taper and the dimensions of the light guide member are selected to provide for preferably no more than two reflections between light entering and leaving the light guide member.

Since lamps such as xenon arc lamps typically have a linear extent which is greater than the size of the region of skin to be treated, the tapered shape of the light guide member allows for efficient collection and concentration of light to be delivered at the output face.

Preferably the light guide member comprises at least one side face which is externally covered with a reflective coating.

Typically the shape of the light guide member will be that of a truncated rectangular pyramid having four side faces which are covered with a reflective coating. Alternative shapes are possible including a truncated circular cylinder having a single side face which is covered with a reflective coating.

The reflective coating is preferably covered with a protective film, preferably of epoxy material.

The use of a reflective coating applied externally obviates the need for cleaning reflective surfaces and such coatings are found to be highly stable in use and resistant to deterioration as a result from high energy use. The input face and the output face are readily accessible for cleaning when necessary and are more hard wearing than reflective surfaces, thereby enhancing the life of the lamp. The provision of the protective film additionally assists in longevity of the reflective coating and prevents damage from external abrasion.

Preferably the light guide member is mounted to the housing by means of a support formed of a plastics material.

A stress relieving plastics material such as PTFE is preferred, thereby surrounding and supporting the light guide member while at the same time tolerating thermal expansion without excessive stress being applied to the light conductive material.

The skin may be presented in contact with a contact face constituted by the output face.

Alternatively the contact face may be constituted by a replaceable/disposable transparent protective layer overlaying the output face. Hygienic use may thereby be ensured by fitting a new protective layer before treating each new patient.

Advantageously the housing comprises means for receiving a filter at a location intermediate the lamp and the light guide member.

A filter such as an ultraviolet absorbing filter may thereby be fitted. Other types of filter may optionally be used, dependent upon the nature of the required treatment.

In the preferred embodiment, the lamp comprises a xenon arc lamp and the light conductive material is fused silica.

The light conductive material may alternatively be formed of high silica borosilicate glass.

The material constituting the light guide member may optionally be doped with a filter material, for example to absorb ultraviolet light without the need for a separate filter.

In a preferred embodiment, the lamp delivers light within the wavelengths 550 to 650 nm.

A preferred embodiment of the present invention will now be described by way of example only and with reference to the accompanying drawings of which;
Figure 1 is a schematic sectioned elevation of apparatus in accordance with the present invention;
Figure 2 is a schematic sectioned plan view of the apparatus of Figure 1;
Figure 3 is a front elevation of the apparatus of preceding Figures;
Figure 4 is a partial sectioned plan view of an alternative apparatus having a ceramic reflector; and
Figure 5 is a sectioned plan view of an alternative light guide member for use in the above apparatus.

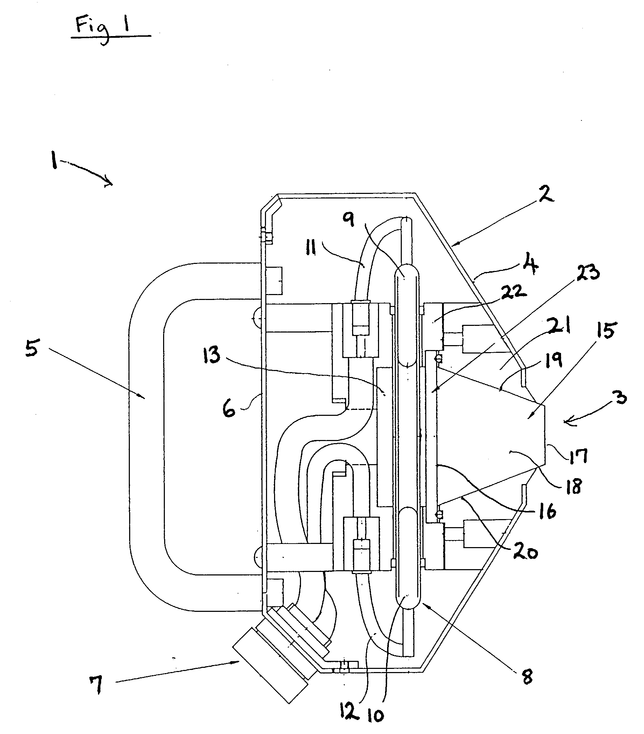

Figure 1 shows an apparatus 1 comprising a housing 2 defining an exit aperture 3 in a conically projecting front wall 4 of the housing. A handle 5 is mounted on a rear wall 6 of the housing 2 and an electrical connector 7 is provided immediately beneath the handle 5.

A xenon arc lamp 8 is mounted in the housing 2 so as to extend linearly in the vertical direction in the normal orientation of the apparatus 1 in use. The arc lamp 8 has electrodes 9 and 10 which are connected to the electrical connector 7 to receive current by means of conductors 11 and 12.

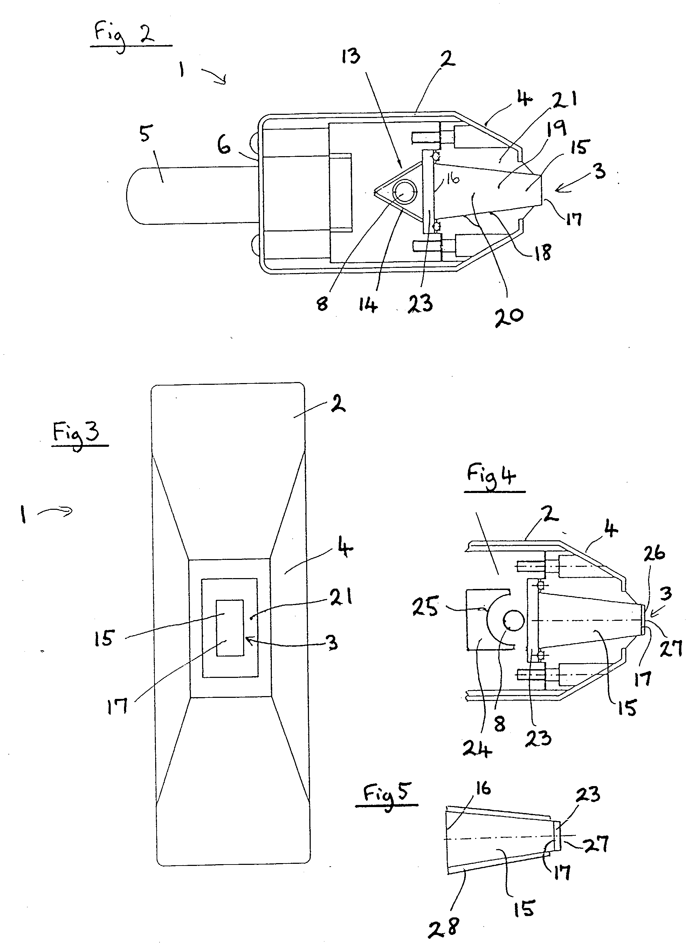

Immediately adjacent the arc lamp 8, first and second reflectors 13 and 14 are mounted so as to reflect light from the lamp in a forward direction towards the exit aperture 3. As shown more clearly in Figure 2, the first and second reflectors 13 and 14 are each constituted by plane silver backed silica mirrors, obliquely mounted rearwardly of the lamp so as to be inclined at about 30° to the forward direction.

Each of the first and second reflectors 13 and 14 is mounted by a centrally located adhesive mounting (not shown) operable to securely mount the reflector while allowing thermal expansion to occur.

A light guide member 15 comprising a block of fused silica is mounted in the housing 2 such that a planar input face 16 extends vertically and adjacent to the arc lamp 8 and a planar output face 17 extends parallel to the input face but located at the exit aperture 3. The light guide member 15 has the shape of a truncated rectangular pyramid which tapers in cross section in the forward direction i.e. towards the exit aperture, the light guide member projecting slightly from the exit aperture such that the output face 17 constitutes the forward extremity of the apparatus.

The light guide member 15 has side faces 18, 19 which are externally silvered to provide internal reflection of light conducted through the light guide member, a silver coating 20 being overlaid with an epoxy protective film (not shown).

The light guide member is clamped within a support 21 formed of polytetrafluoroethylene (PTFE) which extends around the side faces 18 and 19 and is secured to both the front wall 4 of the housing 2 and to a mounting block 22 which supports both the arc lamp 8 and the first and second reflectors 13 and 14.

The mounting block 22 defines a slot for receiving a filter 23 which is removably insertable at a location such that the filter extends parallel to the input face 16 and intermediate the light guide member 15 and the lamp 8.

The filter 23 in the preferred embodiment is an ultraviolet absorbing filter selected to remove ultraviolet light which would otherwise be likely to cause skin damage in use.

The spectrum of the light output from the lamp may be tailored for specific therapeutic uses by suitable choice of filter and/or selection of material for the light guide member 15. The spectrum may for example extend from 500 nm to a wavelength at the limit of transmission in the infrared of the light conductive material of the light guide member 15 in order to specifically include infra red radiation as part of the therapy.

In the above embodiment, the reflectors 13 and 14 may alternatively be comprised by ceramic diffuse reflective plates. A further alternative is illustrated with reference to Figure 4 in which a reflector 24 is constituted by a ceramic reflector unitarily formed of a block of ceramic material having a shaped reflective surface 25 extending around the rear of the arc lamp 8 so as to reflect light forwardly into the light guide member 15. In Figure 4 the shape of the reflective surface is semicircular when viewed in axial projection. The reflector 24 is formed from a block of porous aluminium oxide with the reflective surface 24 being formed by a thin glaze coating. The reflective surface 25 is thereby rendered hard wearing and easy to clean, the reflector 24 thereby providing a stable long lasting reflective structure requiring minimal maintenance.

The embodiment of Figure 4 also includes a disposable protective layer 26 overlaying the output face 17 to define a contact face 27 which can be renewed before each use by changing the layer 26.

The protective layer 26 may be a transparent plastic film releasably covering the output face 17 or may be a glass or silica plate mounted in a suitable mounting (not shown) of the housing 2.

The light conductive material of the light guide member 15 may alternatively be borosilicate glass. The light conductive material may optionally be doped with a filter material, for example to absorb ultraviolet light, thereby obviating the need for a separate filter 23. This has the advantage that absorption of light by the filter material is distributed throughout the bulk of the light guide member 15, thereby reducing temperature rise and associated stress in components of the apparatus 1.

Further alternatives include the embodiment illustrated with reference to Figure 5 using corresponding reference numerals to those of preceding Figures where appropriate. Figure 5 shows a light guide member 15 having the same tapered configuration as the light guide member of preceding embodiments but having a filter 23 sealed in face to face contact with an output face 17 of the light guide member. The light guide member 15 is encased in an aluminium casing 28 and is removably received in apparatus of the type shown in Figures 1 or 2 or Figure 4. The light guide member 15 and the filter 23 are in this instance each selected to provide appropriate filtering properties and it is intended that the apparatus would in this instance be supplied with a number of light guide members, each combination of light guide member and filter constituting a filtering unit providing an individual filtering regime to enable the user to select appropriate filtering characteristics to individual user requirements. To change from one filtering regime to another, this would require removal of the existing filtering unit and replacement with another.

The light guide member 15 of Figure 5 is sealed to the filter 23 in a manner which prevents ingress of material between the filter and the light guide member. This is particularly important where the filter 23 provides the contact face 27 and is therefore likely to be exposed to lubricating gel in use.

Lamps other than xenon arc lamps may be utilised, for example mercury arc lamps.

Cooling of the lamp 8 is provided in the above embodiments by means of an air compressor which delivers a high flow of cooling air in proximity with the lamp.

The silver coating 20 referred to with reference to the embodiments of Figures 1 to 4 may be omitted, as in the case of the embodiment of Figure 5.

Typically the lamp 8 will generate light pulses of about 400J per pulse, delivering 30J at the contact face 26. For a typical contact face 26 having a surface area of 2cm² the lamp 8 therefore delivers 15J per cm². Typical operation of the lamp 8 would provide one pulse every 10 seconds.

The term "light" used throughout the specification is to be understood as including non-visible infrared and ultraviolet radiations generated by the above mentioned lamps.

## Claims

1. Apparatus (1) for use in therapeutic skin treatment comprising a housing (2) defining an exit aperture (3), an arc lamp (8) mounted in the housing and operable to deliver light via the exit aperture, a light guide member (15) mounted in the housing and comprising a block of light conductive material having an input face (16) disposed adjacent to the lamp and an output face (17) at the exit aperture whereby in use an area of skin presented to the output face receives light from the lamp.

2. Apparatus as claimed in Claim 1 wherein the light guide member is tapered in cross section towards the output face such that in use light from the lamp is concentrated before emerging from the output face.

3. Apparatus as claimed in any preceding claim wherein the light guide member comprises at least one side face (18, 19) which is externally covered with a reflective coating (20).

4. Apparatus as claimed in Claim 3 wherein the reflective coating is covered with a protective film of epoxy material.

5. Apparatus as claimed in any preceding claim wherein the light guide member is mounted to the housing by means of a support (21) formed of a plastics material.

6. Apparatus as claimed in Claim 5 wherein the plastics material is polytetrafluoroethylene(PTFE).

7. Apparatus as claimed in any preceding claim wherein the light guide member is provided with a replaceable transparent protective layer (26) overlaying the output face and defining a contact face (27) which in use is presented in contact with the area of skin.

8. Apparatus as claimed in any preceding claim wherein the housing comprises means for receiving a filter (23) at a location intermediate the lamp and the light guide member.

9. Apparatus as claimed in any of Claims 1 to 7 wherein a filter (23) is secured in sealed relationship to the output face of the light guide member.

10. Apparatus as claimed in Claim 9 wherein the light guide member and the filter in combination constitute a replaceable filtering unit whereby a range of predetermined filtering characteristics may be obtained in use by selectively inserting a filtering unit selected from a set of filtering units having different filtering characteristics.

11. Apparatus as claimed in any of Claims 8 to 10 wherein the filter is operable to absorb ultraviolet radiation.

12. Apparatus as claimed in any preceding claim wherein the light conductive material is fused silica.

13. Apparatus as claimed in any preceding claim wherein the light conductive material includes a constituent operable to absorb ultraviolet radiation whereby the light guide member functions as an ultraviolet filter.

14. Apparatus as claimed in any preceding claim wherein the lamp is operable to deliver light within the wavelengths 550 to 650 nm.

15. Apparatus as claimed in any preceding claim comprising a ceramic reflector (24) adjacent a rear face of the lamp and operable to reflect light towards the light guide member.

16. Apparatus as claimed in any preceding claim comprising an air compressor operable to supply a flow of cooling air to the lamp.
